# EUROPEAN PATENT APPLICATION

(11) **EP 4 656 156 A1**
(43) Date of publication of application: **03.12.2025**
(21) Application number: 25178934.3
(22) Date of filing: 27.05.2025
(51) Int. Cl.: A61B 90/00, A61B 34/10, A61B 34/00, G01L 1/00, G06F 3/00

(54) **SENSING DEVICE AND CONTROL SYSTEM INCLUDING THE SAME**

(30) Priority: 27.05.2024 KR 20240068722
(71) Applicant: Endorobotics Co., Ltd., Seoul 02841 (KR)
(72) Inventor: SHIN, Woo Cheol, 02577 Seoul (KR); KIM, Kyung Nam, 02577 Seoul (KR); KO, Yeon Ho, 02577 Seoul (KR); WON, Seong Hyeon, 02577 Seoul (KR); KIM, Chan Woo, 02577 Seoul (KR); LEE, Yong Jung, 02577 Seoul (KR)
(74) Representative: Herrmann, Daniel

(57) **Abstract**

A sensing device is provided. The sensing device according to an aspect of the present disclosure includes a housing including a first side wall member arranged in an up-down direction with respect to a bottom surface; a first handle formed in a closed curve shape, at least a portion of which is exposed to an outside of the housing and placed on a predetermined first surface, and coupled to one side of the first side wall member so as to be capable of orbital movement along an extension direction of the closed curve at a predetermined location on the closed curve; a first rotating body configured to rotate around a predetermined first rotation axis in conjunction with the orbital movement of the first handle; a support member coupled to the housing, and configured to support the first handle such that the first handle maintains a fixed position at the predetermined location; and a first sensor configured to sense information on a rotational movement of the first rotating body.

## Description

### CROSS-REFERENCE TO RELATED APPLICATION(S)

This application claims priority to and the benefit of Korean Patent Application No. 10-2024-0068722, filed on May 27, 2024, the disclosure of which is incorporated herein by reference in its entirety.

### TECHNICAL FIELD

The present disclosure relates to a sensing device and a control system including the same, and more specifically to a sensing device for remotely controlling a passive member by imitating a surgical operation and a control system including the same.

### RELATED ART

An endoscope is a device designed to insert a machine into the body to observe lesions in organs that cannot be directly observed without surgery or an autopsy. Recently, various types of surgical tools have been designed to perform procedures on the inside of organs without cutting the patient's body.

Generally, a passive member in the form of a thin and long tube, such as a catheter or guide wire, is used to perform procedures on the inside of a patient's organ.

In this case, a radiological marker, such as an X-ray marker, may be used to accurately determine the location of the catheter or guide wire inserted into the patient's body, but this may expose the doctor to radiation during the surgery.

In order to solve this problem, a control system that can remotely control surgical tools such as catheters or guide wires is being developed.

**In** order for a control system that can remotely control surgical tools to operate, a sensing device that can sense a user's input signal, a communication module that transmits the user's input signal, and a surgical tool that can be controlled by receiving an input signal from the communication module are required.

Since surgical tools such as catheters or guide wires are inserted into the patient's organs, they must be precisely controlled.

Nevertheless, existing sensing devices are operated by buttons or joysticks, and thus, there is a problem in that there is a large gap between the sensation felt when actually using the surgical tool and the sensation felt during the process of inputting a signal to the sensing device.

Since the success of the surgery can be directly related to the patient's life, such differences in sensation felt during the manipulation process can have a significant impact on the success of the surgery and the patient's safety.

In addition, although surgical tools such as catheters or guide wires that are inserted into the patient's organs can be formed in the form of thin and long tubes, existing sensing devices are formed in a linear structure, and thus, there is a limitation to the displacement that can be manipulated in one direction.

Accordingly, control systems using existing sensing devices have limitations in continuous one-way control operations.

For example, in control systems using existing sensing devices, after moving the sensing device in one direction by a certain distance, a reciprocating movement to return the device to its original position for additional movement is essential, and thus, there has been a problem in that the manipulation process is unnatural.

Accordingly, there is a need for a sensing device and a control system including the same that can control a surgical tool at a distance with a feeling similar to manipulating an actual surgical tool while allowing continuous manipulation in one direction.

### SUMMARY

The present disclosure has been devised to solve the above problems, and an object of the present disclosure is to provide a sensing device that can be operated through a movement that is similar to a movement using an actual surgical tool, and a control system including the same.

Another object of the present disclosure is to provide a sensing device that can be continuously operated in one direction without displacement limitation through a closed curve-shaped operating part structure, and a control system including the same.

The problems of the present disclosure are not limited to the problems mentioned above, and other problems that are not mentioned will be clearly understood by those skilled in the art to which the present disclosure belongs from the description below.

According to an aspect of the present disclosure, provided s a sensing device, including a housing including a first side wall member arranged in an up-down direction with respect to a bottom surface; a first handle formed in a closed curve shape, at least a portion of which is exposed to an outside of the housing and placed on a predetermined first surface, and coupled to one side of the first side wall member so as to be capable of orbital movement along an extension direction of the closed curve at a predetermined location on the closed curve; a first rotating body configured to rotate around a predetermined first rotation axis in conjunction with an orbital movement of the first handle; a support member coupled to the housing, and configured to support the first handle such that the first handle maintains a fixed position at the predetermined location; and a first sensor configured to sense information on a rotational movement of the first rotating body.

In this case, the sensing device may further include a second handle formed to be rotatable at a fixed position; a second rotating body configured to rotate around a predetermined second rotation axis in conjunction with a rotational movement of the second handle; and a second sensor configured to sense information about a rotational movement of the second rotating body.

In this case, the first surface may be a plane, and the second rotation axis may be parallel to the first surface or belongs to the first surface.

In this case, the second handle may be formed in a pipe shape including a first hole penetrating the second handle in a direction parallel to the second rotation axis, and at least a portion of the first handle may penetrate the first hole.

In this case, the sensing device may further include a third rotating body configured to share a rotation axis with the second handle and be connected to a lower part of the second handle and rotate integrally with the second handle, wherein the second rotating body is rotated by a rotational force of the third rotating body.

In this case, the third rotating body may rotate around a third rotation axis parallel to the second rotation axis, a second power transmission member in a belt shape may be connected to a peripheral part of the third rotating body and a peripheral part of the second rotating body, and the second power transmission member may transmit a rotational force of the third rotating body to the second rotating body.

In this case, the second sensor may be coupled to the second rotation axis, and sense information about a rotational movement of the second rotating body transmitted through the second rotation axis.

In this case, the sensing device may further include a plate-shaped working surface configured to cross the first side wall member at a predetermined height, wherein the working surface may be formed with a second hole penetrated by the second handle, and to which the second handle is coupled.

In this case, the second rotation axis may be received at one end part of the working surface, and a receiving groove for supporting the second rotation axis may be formed by being recessed in a direction perpendicular to an axial direction of the second rotation axis.

In this case, the first surface may be a plane, and the first rotating body may be arranged on the first surface, wherein a peripheral part of the first handle and an outer surface of the first rotating body may be connected together to a first power transmission member in a belt shape, and wherein the first power transmission member may transmit a driving force according to an orbital movement of the first handle to the first rotating body to rotate the first rotating body.

In this case, the first sensor may be coupled to a first rotation axis that becomes a center of rotation of the first rotating body, wherein the first sensor may include a reaction force providing means for rotating the first rotation axis, and wherein the first sensor may be capable of transmitting a force toward the first handle through the first power transmission member by rotating the first rotating body.

In this case, the first sensor may sense a torque applied to the first rotation axis.

In this case, a peripheral part of the first handle and an outer surface of the first rotating body may be arranged to be in contact with each other.

In this case, the first rotating body may be rotated by a frictional force generated at a contact part of the first rotating body and the first handle according to an orbital movement of the first handle.

In this case, the first handle may include gear teeth that are continuously provided along a peripheral part, and the first rotating body may be formed in a gear shape that meshes with the gear teeth such that the first rotating body that meshes with the first handle rotates according to an orbital movement of the first handle.

In this case, the first handle may be formed in a belt shape, and an outer surface of the first rotating body may be formed to contact an inner surface of the first handle such that the first rotating body is rotated according to an orbital movement of the first handle.

In this case, the first sensor may be coupled to a first rotation axis that becomes a center of rotation of the first rotating body, the first sensor may include a reaction force providing means for rotating the first rotation axis, and the first sensor may be capable of transmitting a force toward the first handle by rotating the first rotating body.

In this case, the first sensor may sense a torque applied to the first rotation axis.

In this case, the support member may be formed by a plurality of pulleys that support an inner or outer surface of the first handle, and rotational axes of a plurality of support members may be each fixed at one end part to the first side wall member.

In this case, the second sensor may include a reaction force providing means for rotating the second rotation axis, and the second sensor may be capable of transmitting a force toward the second handle through the second power transmission member by rotating the second rotational body.

In this case, the second sensor may sense a torque applied to the second rotation axis.

In this case, the first handle may be formed in a circular ring shape.

According to another aspect of the present disclosure, provided is a control system, including the sensing device; a passive member configured to be controlled according to information sensed by the sensing device; a communication module configured to be capable of sending and receiving electrical signals from the sensing device and the passive member; and a controller configured to control the sensing device and the passive member through the communication module, wherein the controller controls a linear movement of the passive member according to orbital movement information of the first handle sensed by the first sensor, and controls a rotational movement of the passive member according to rotational movement information of the second handle sensed by the second sensor.

In this case, the passive member may include a pressure sensor that senses a pressure, and the controller may control the first rotating body or the second rotating body to transmit a reaction force corresponding to a pressure sensed by the pressure sensor to the first handle or the second handle.

According to the above configuration, the sensing device according to one aspect of the present disclosure and the control system including the same can remotely control a surgical tool with a motion that is similar to the motion of manipulating an actual surgical tool by manipulating a linear movement of the surgical tool by turning a closed-curve shaped handle and manipulating a rotational movement of the surgical tool by turning a cylindrical handle surrounding a closed-curve handle.

The sensing device according to another aspect of the present disclosure and the control system including the same can control a linear movement through a closed-curve shaped handle, thereby controlling the linear movement with continuous motion without displacement restrictions.

The sensing device according to still another aspect of the present disclosure and the control system including the same are provided such that the handle in charge of a rotational movement surrounds the handle in charge of a linear movement, and thus, it is easy to control a linear movement and a rotational movement at the same time.

The sensing device according to still another aspect of the present disclosure and the control system including the same can transmit a reaction force applied to the surgical tool to the handle by adopting a motor and a torque sensor, and thus, the reaction force that can be applied when manipulating an actual surgical tool can be similarly transmitted.

The sensing device according to still another aspect of the present disclosure and the control system including the same can allow a user to perform long-term remote control with minimal fatigue, by positioning a handle on a working surface.

The effects of the present disclosure are not limited to the above-described effects, and should be understood to include all effects that can be inferred from the configurations of the invention described in the detailed description or claims of the present disclosure.

### BRIEF DESCRIPTION OF THE DRAWINGS

FIG. 1 is a perspective view of a sensing device according to an embodiment of the present disclosure.
FIG. 2 is a perspective view illustrating a sensing device according to an embodiment of the present disclosure viewed from another direction.
FIG. 3 is a left side view of a sensing device according to an embodiment of the present disclosure.
FIG. 4 is a plan view of a sensing device according to an embodiment of the present disclosure.
FIG. 5 is a front view of a sensing device according to an embodiment of the present disclosure.
FIG. 6 is a rear view of a sensing device according to an embodiment of the present disclosure.
FIG. 7 is a structural diagram of a sensing device according to an embodiment of the present disclosure.
FIG. 8 is an exploded perspective view of a sensing device according to an embodiment of the present disclosure.
FIG. 9 is a cross-sectional view of a sensing device according to an embodiment of the present disclosure taken along line I-I' of FIG. 1.
FIG. 10 is a drawing illustrating components of a sensing device excluding a housing according to an embodiment of the present disclosure.
FIG. 11 is a drawing illustrating some configurations related to the rotational movement of a second handle of a sensing device according to an embodiment of the present disclosure.
FIG. 12 is a drawing illustrating some configurations related to the rotational movement of a first handle of a sensing device according to an embodiment of the present disclosure.
FIG. 13 is a drawing illustrating a first modified example of some configurations related to the rotational movement of a first handle of a sensing device according to an embodiment of the present disclosure.
FIG. 14 is a drawing illustrating a second modified example of some configurations related to the rotational movement of a first handle of a sensing device according to an embodiment of the present disclosure.
FIG. 15 is a perspective view of a sensing device according to another embodiment of the present disclosure.
FIG. 16 is a drawing illustrating some configurations related to the rotational movement of a first handle of a sensing device according to another embodiment of the present disclosure.
FIG. 17 is a structural diagram of a control system including a sensing device according to an embodiment of the present disclosure.

### DETAILED DESCRIPTION OF THE EMBODIMENTS

Hereinafter, with reference to the attached drawings, embodiments of the present disclosure will be described in detail so that those skilled in the art can easily practice the invention. The present disclosure may be implemented in various different forms and is not limited to the embodiments described herein. In the drawings, parts that are not related to the description are omitted in order to clearly explain the present disclosure, and the same reference numerals are assigned to identical or similar components throughout the specification.

The words and terms used in the present specification and claims should not be interpreted as having a limited or dictionary meaning, but should be interpreted as having a meaning and concept that conforms to the technical idea of the present disclosure according to the principle that the inventor can define terms and concepts in order to explain his or her invention in the best way.

In the present specification, the terms "include" or "have" are intended to explain the presence of a feature, number, step, operation, component, part or combination thereof described in the specification, and should be understood as not excluding in advance the possibility of the presence or addition of one or more other features, numbers, steps, operations, components, parts or combinations thereof.

In order to clearly express the features of the configuration in the drawing, the thickness and size are exaggerated, and the thickness and size of the configuration illustrated in the drawing are not necessarily the same as the actual ones.

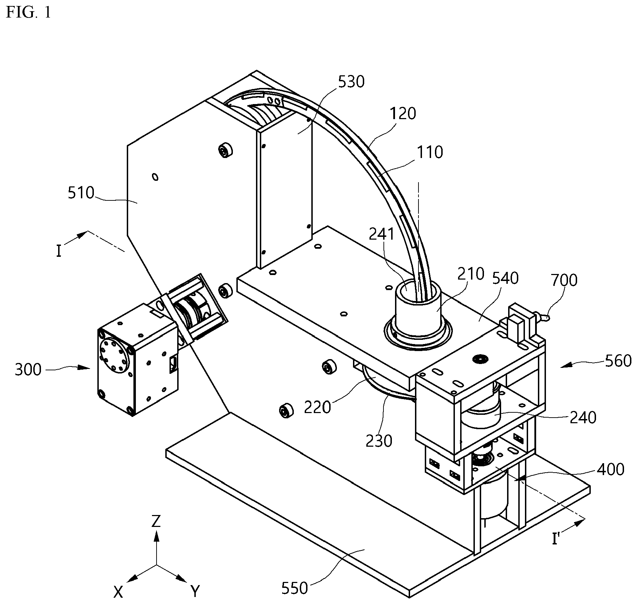

In the following description of the drawing, each direction is defined based on the coordinate axes illustrated in FIG. 1. More specifically, the positive direction of the z-axis is defined as an upper side, and the negative direction of the z-axis is defined as a lower side. The positive direction of the y-axis is defined as a front side, and the negative direction of the y-axis is defined as a back side. The positive direction of the x-axis is defined as a left side, and the negative direction of the x-axis is defined as a right side.

In the following description, the description of some components may be omitted in order to clearly express the features of the present disclosure.

The singular expression includes the plural expression unless it is clearly meant differently in context.

A sensing device according to an embodiment of the present disclosure is a sensing device that senses a user's movement signal to remotely control a passive member by imitating the user's movement.

In this case, the passive member may be a surgical tool such as a catheter or a guide wire, but the passive member that the sensing device of the present disclosure can use is not limited thereto.

For example, the passive member controlled by the sensing device according to an embodiment of the present disclosure is not limited to a surgical tool, and it may be a thin and long tube-shaped object, and other types of objects may also be used as the passive member.

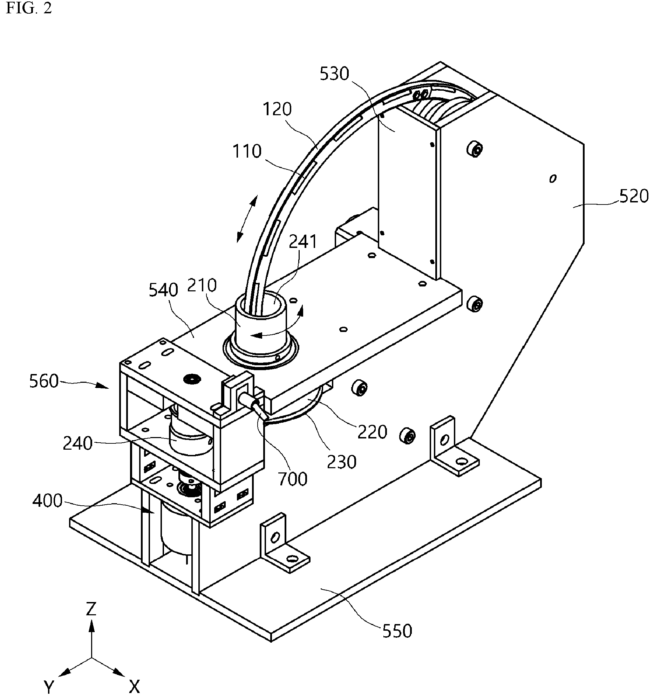

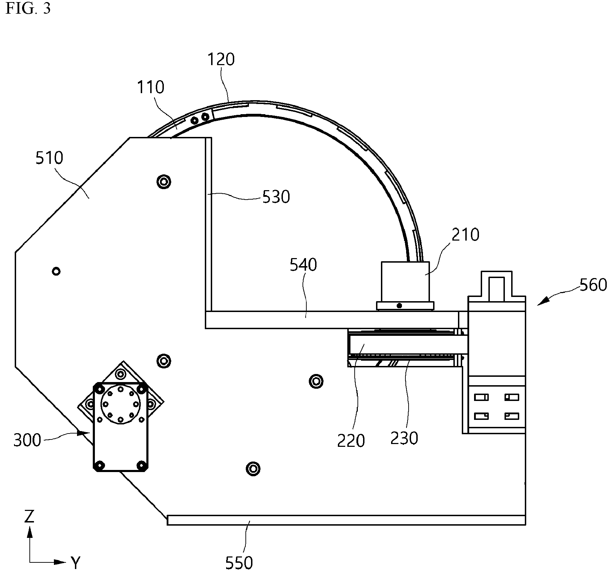

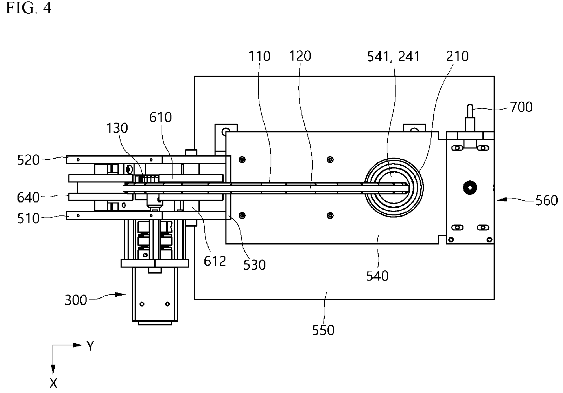

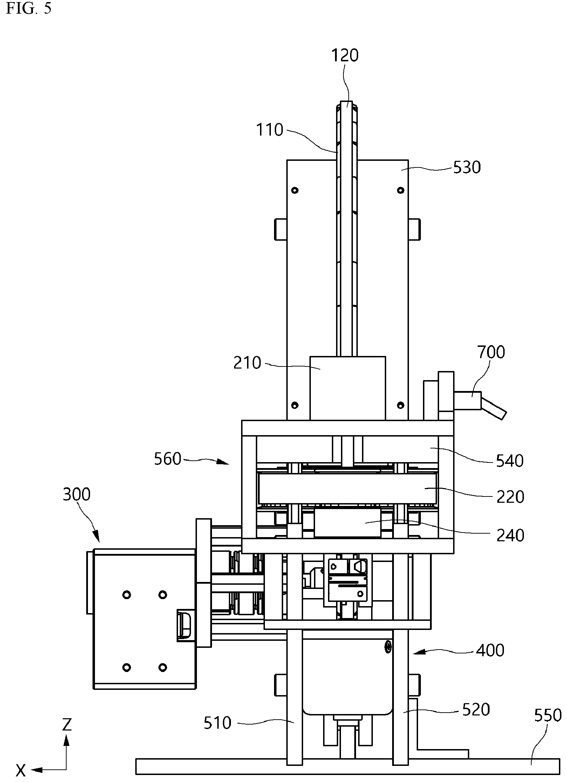

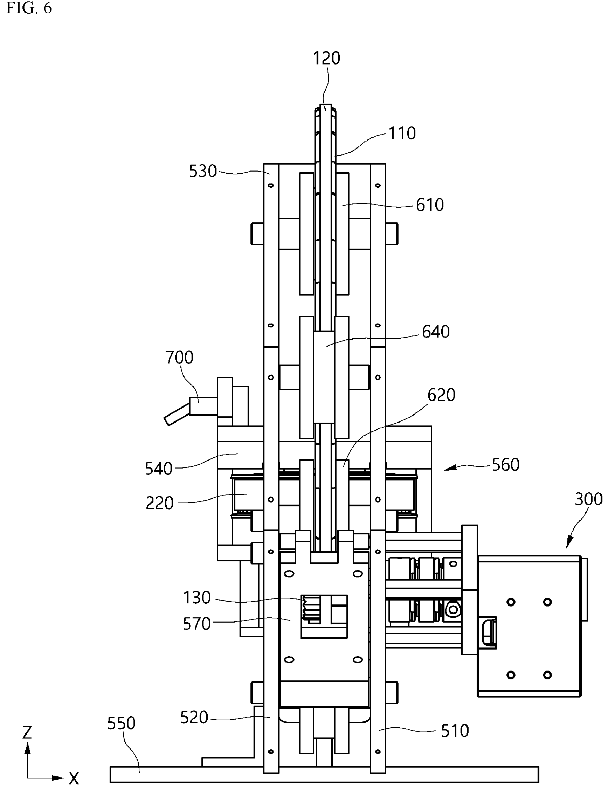

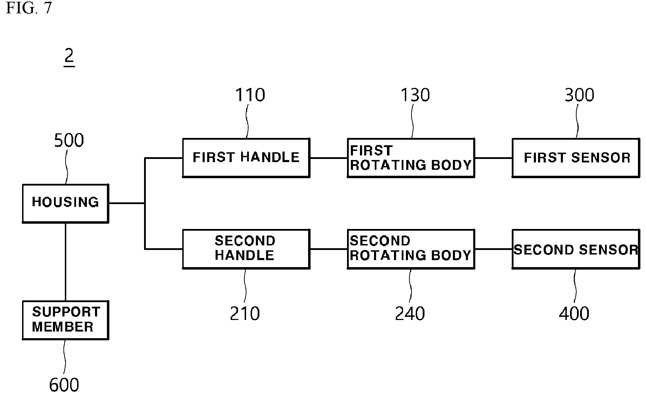

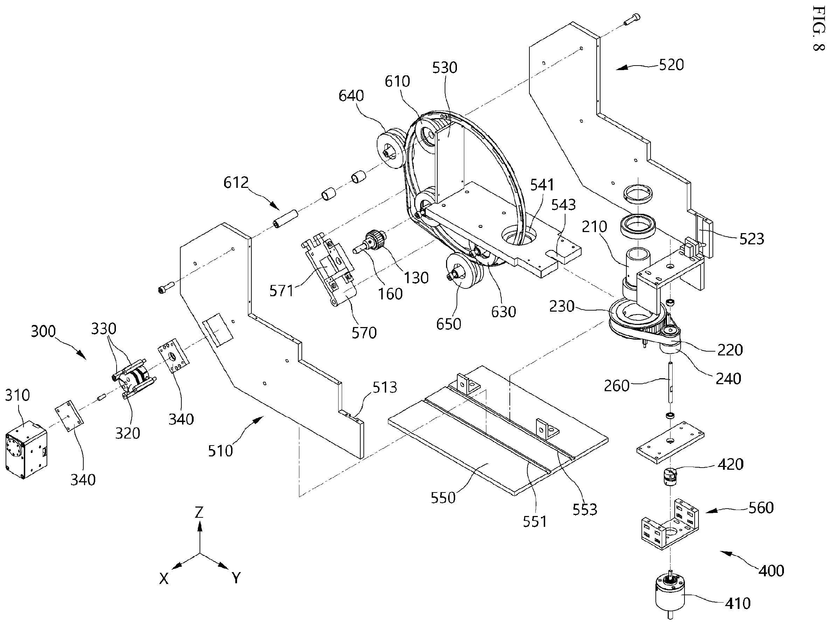

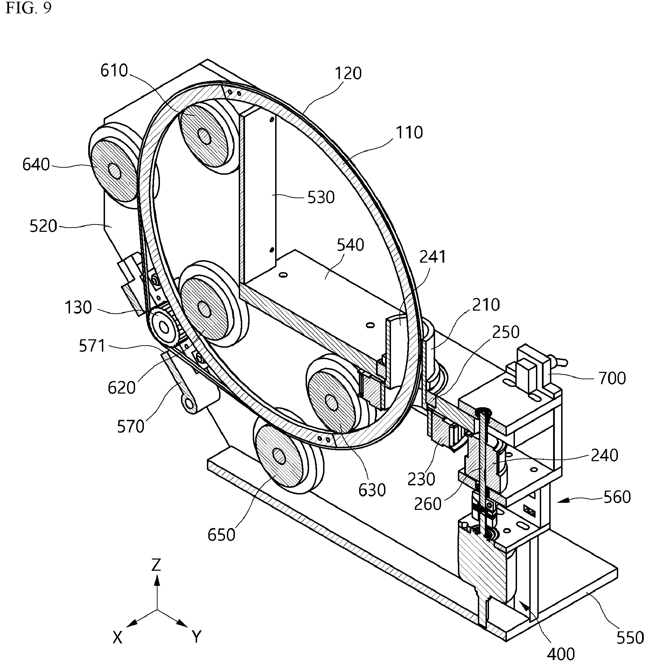

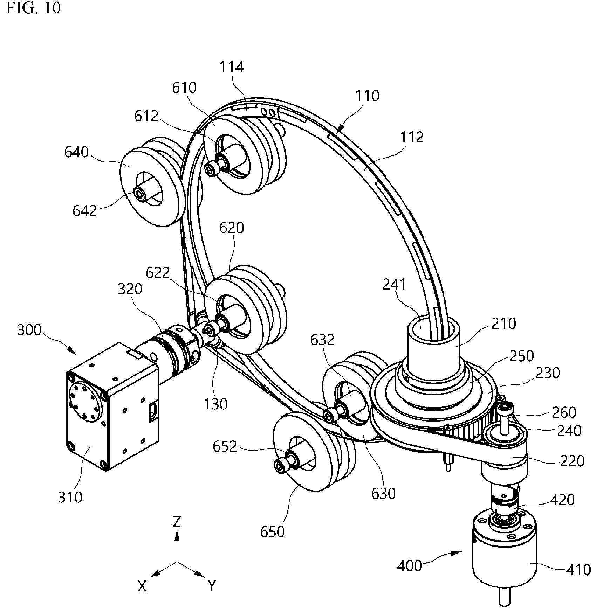

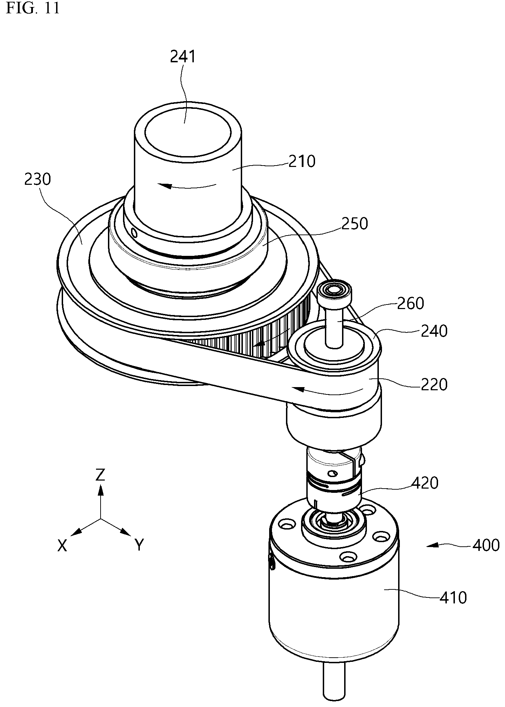

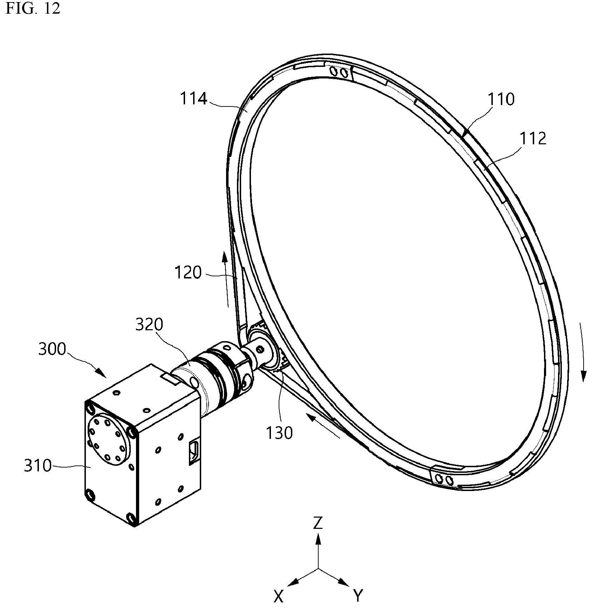

FIG. 1 is a perspective view of a sensing device according to an embodiment of the present disclosure. FIG. 2 is a perspective view illustrating a sensing device according to an embodiment of the present disclosure viewed from another direction. FIG. 3 is a left side view of a sensing device according to an embodiment of the present disclosure. FIG. 4 is a plan view of a sensing device according to an embodiment of the present disclosure. FIG. 5 is a front view of a sensing device according to an embodiment of the present disclosure. FIG. 6 is a rear view of a sensing device according to an embodiment of the present disclosure. FIG. 7 is a structural diagram of a sensing device according to an embodiment of the present disclosure. FIG. 8 is an exploded perspective view of a sensing device according to an embodiment of the present disclosure. FIG. 9 is a cross-sectional view of a sensing device according to an embodiment of the present disclosure taken along line I-I**'** of FIG. 1. FIG. 10 is a drawing illustrating components of a sensing device excluding a housing according to an embodiment of the present disclosure. FIG. 11 is a drawing illustrating some configurations related to the rotational movement of a second handle of a sensing device according to an embodiment of the present disclosure. FIG. 12 is a drawing illustrating some configurations related to the rotational movement of a first handle of a sensing device according to an embodiment of the present disclosure.

A sensing device 2 according to an embodiment of the present disclosure may include a first handle 110 and a first rotating body 130 for controlling one-way movement of a passive member, a support member 600 and a housing 500 for supporting the first handle 110, and a first sensor 300.

In addition, the sensing device 2 according to an embodiment of the present disclosure may include a second handle 210 and a second rotating body 240 for controlling the rotational movement of a passive member, a housing 500 for supporting the second handle 210, and a second sensor 400.

In this case, the housing 500 of the sensing device 2 according to an embodiment of the present disclosure may include a bottom surface 550, a first side wall member 510 arranged in an up-down direction with respect to the bottom surface 550, a second side wall member 520 that may be arranged parallel to the first side wall member 510, a third side wall member 530 arranged toward a front side, and a working surface 540 arranged toward an upper side.

In this case, the first handle 110 of the sensing device 2 according to an embodiment of the present disclosure may be formed in a closed curve shape that is placed on a predetermined first surface and be supported by a support member 600 fixed to the first side wall member 510.

In this case, the first handle 110 may be connected to the first side wall member 510 such that orbital movement is possible along an extension direction of the closed curve at a predetermined position on the closed curve forming the first handle 110.

More specifically, referring to FIGS. 1 to 10, the first handle 110 is formed in the form of a closed curve, and it may be connected to a side surface of the first side wall member 510 by a support member including a first support member 610, a second support member 620 and a third support member 630.

Accordingly, the entire first handle 110 is arranged at a constant position in space, and each component part constituting the first handle 110 may move from one position on the closed curve to another position on the closed curve along a path of the closed curve forming the first handle 110.

Accordingly, each component part constituting the first handle 110 may perform an orbital movement along the path of the closed curve.

The support member may be composed of five or more support members, including a first support member 610, a second support member 620, a third support member 630, a fourth support member 640 and a fifth support member 650, as illustrated.

Accordingly, the first handle 110 may be supported in various directions, thereby fixing a spatial position of the first handle 110.

However, the structure and number of the support members are not limited thereto, and are not limited as long as they have a shape, structure and number that can fix the position of the first handle 110 in space.

For example, the support members may be composed of only three to support different directions, or may be provided with six or more.

In addition, as illustrated in the drawings, the support member may be formed in the shape of a plurality of pulleys including a first support member rotation axis 612 that becomes a rotation center axis of the first support member 610, a second support member rotation axis 622 that becomes a rotation center axis of the second support member 620, a third support member rotation axis 632 that becomes a rotation center axis of the third support member 630, a fourth support member rotation axis 642 that becomes a rotation center axis of the fourth support member 640, and a fifth support member rotation axis 652 that becomes a rotation center axis of the fifth support member 650, as illustrated.

However, it is not necessarily limited thereto, and the support member may be formed in a rail shape or a gear shape, and is not limited to being formed in other structures.

For example, as illustrated in FIGS. 1 to 10, when the first handle 110 is formed in a circular closed curve shape, the first handle 110 may perform a rotational movement centered on a rotation axis perpendicular to a first surface on which the first handle 110 is placed at a fixed position.

Accordingly, each component constituting the first handle 110 may perform an orbital movement centered on a rotation axis located at the center of the circular first handle 110 perpendicular to the first surface.

In this way, each component part of the first handle 110 may perform an infinite orbital movement along an extension direction of the closed curve constituting the first handle 110 without any limitation on the distance of movement.

As a result, even if the length of a periphery of the closed curve forming the first handle 110 is shorter than the length of the passive member or the length of a periphery of the closed curve is shorter than the displacement to which the passive member is to be moved, the first handle 110 may perform an infinite orbital movement, and thus, it is possible to manipulate the passive member in one direction without any limitation on displacement.

For example, if the passive member is provided as a catheter and the first handle 110 is provided in a circular shape, when inserting the catheter into an organ by a displacement corresponding to twice the circumference of the closed curve forming the first handle 110, the catheter may be inserted to a target position by simply turning the first handle 110 twice.

In the case of existing sensing devices that are not provided with a closed curve shape, the passive member could be manipulated by repeatedly moving the sensing device a predetermined distance, realigning the sensing device, and moving the same again a predetermined distance. However, the sensing device 2 according to an embodiment of the present disclosure may be manipulated continuously without such limitations.

In addition, even if the first handle 110 is formed in a closed curve shape, when manipulating the handle 110, it is not necessary to manipulate the first handle 110 along the closed curve shape, and a desired part of the first handle 110 may be manipulated at a location in space.

Accordingly, the first handle 110 may be manipulated by moving a specific part on the first handle 110 in a tangential direction of the first handle 110.

More specifically, for example, when the first handle 110 is formed in a circular ring shape, the user does not have to hold the first handle 110 and manipulate the first handle 110 along a circular orbit in order to manipulate the first handle 110.

The user may manipulate the first handle 110 by repeatedly manipulating only a specific part of the first handle 110 that enters the second handle 210 as seen in FIG. 1 such that the first handle 110 is pulled upward or pushed downward into the second handle 210.

Accordingly, the user may manipulate the first handle 110 with a movement similar to manipulating an actual passive member.

As illustrated in the drawings, the first handle 110 may be formed in a circular ring shape, and accordingly, the first surface where the first handle 110 is arranged may be formed as a single plane.

However, it is not necessarily limited thereto, and the shape of the first handle 110 may be formed in a closed curve shape other than a circle, and the first surface may also be formed as a curved surface, not necessarily a plane.

In addition, the first handle 110 may be formed as a single closed-curve-shaped member, and may also be formed in a form in which a first part 112 and a second part 114 are combined through a coupling part 116 as illustrated in the drawings.

The configuration of the first handle 110 is not limited thereto, and it may also be formed in a form in which additional components are coupled, such as a third part and a fourth part, which are not illustrated.

The first handle 110 may directly or indirectly rotate the first rotating body 130 provided on one side of the first handle 110 to transmit information about an orbital movement of the first handle 110 to the first sensor 300.

A first power transmission member 120 for transmitting a force to the first rotating body 130 may be connected to the first handle 110 of the sensing device 2 according to an embodiment of the present disclosure.

The first power transmission member 120 of the sensing device 2 according to an embodiment of the present disclosure may be formed in a belt shape, and may be arranged to be in contact with a peripheral part of the first handle 110 and an outer surface of the first rotating body 130.

In this case, the first handle 110 has a groove formed along a peripheral direction into which the first power transmission member 120 can be mounted such that the first power transmission member 120 may be seated in the groove formed in the first handle 110.

The first rotating body 130 is a rotating body provided on one side of the first handle 110, and as illustrated in FIGS. 8 to 10, it may rotate around a first rotation axis 160 perpendicular to the first side wall member 510.

The direction of the first rotation axis 160, which is a rotation center axis of the first rotating body 130, the position where the first rotating body 130 is arranged, and the shape of the first rotating body 130 are not limited as long as the first rotating body 130 can transmit a force from the first handle 110 to the first sensor 300.

The first power transmission member 120 of the sensing device 2 according to an embodiment of the present disclosure may contact the first rotating body 130 so as to surround at least a portion of the outer surface of the first rotating body 130.

At the same time, the first power transmission member 120 may contact the first handle 110 so as to surround at least a portion of the outer surface of the first handle 110.

Accordingly, when the first handle 110 orbits in one direction, the first handle 110 may cause the first power transmission member 120 to orbit in the same direction, and accordingly, the first power transmission member 120 may rotate the first rotating body 130 clockwise or counterclockwise.

For example, referring to FIG. 12, when the first handle 110 is formed in a circular ring shape, if the first handle 110 orbits in a clockwise direction, the first power transmission member 120 connected to the first handle 110 may also orbit in a clockwise direction as a whole.

In this case, the first rotating body 130 connected to the first power transmission member 120 may also be rotated clockwise by the first power transmission member 120.

The first power transmission member 120 may be provided as a belt, but is not necessarily limited thereto, and may also be provided as a chain, and is not limited as long as it can transmit the driving force transmitted through the first handle 110 to the first rotating body 130.

A first sensor 300 may be arranged at one end of a longitudinal direction of the first rotation axis 160, which becomes a rotational center axis of the first rotating body 130.

The first sensor 300 may sense information about a rotational movement of the first rotating body 130.

To this end, the first sensor 300 may be provided as an encoder that can collect information about the rotational movement, such as the rotation amount, angular velocity and torque of the first rotating body 130.

Referring to FIGS. 1 to 10, the first sensor 300 may include a first sensor body 310, a rotating part 320, a frame 330 and a fixing part 340.

In this case, the first rotating body 130 may be relatively arranged at a back side of the sensing device 2 according to an embodiment of the present disclosure.

Accordingly, a back housing 570 that is capable of supporting and covering the first rotating body 130 may be arranged at a back side of the sensing device 2 according to an embodiment of the present disclosure.

In this case, a separate third hole 571 may be formed in the back housing 570 at a part where there is a possibility of physical interference with the first rotating body 130 such that the first rotating body 130 can rotate smoothly, thereby preventing physical interference.

The rotating part 320 of the first sensor 300 is coupled to a first rotation axis 160 and may rotate together with the first rotation axis 160.

As the first rotation axis 160 rotates together with the first rotating body 130, the rotating part 320 rotates integrally with the first rotating body 130, and information on the rotational movement of the first rotating body 130 may be sensed by an encoder provided in the first sensor body 310.

In this case, an encoder provided with a reaction force providing means may be placed in the first sensor body 310.

Accordingly, the first sensor 300 may transmit a predetermined reaction force toward the first handle 110 by the reaction force providing means provided in the encoder of the first sensor 300.

More specifically, by using the reaction force providing means provided in the encoder of the first sensor 300, the first sensor 300 may transmit a predetermined torque to the first rotation axis 160.

The torque transmitted to the first rotation axis 160 may be transmitted to the first rotating body 130, and the torque transmitted to the first rotating body 130 may be transmitted to the first handle 110 through the first power transmission member 120, or, if the first power transmission member 120 is not provided, the first rotating body 130 may directly transmit the torque to the first handle 110.

Accordingly, when the passive member comes into contact with an obstacle in an organ or receives an external force due to the surrounding environment, the corresponding external force may be transmitted to the first handle 110. Accordingly, it is possible to implement a reaction force that can be felt when a user directly grips and manipulates the passive member.

In this case, the first sensor 300 may include a torque sensor that is capable of sensing a torque applied to the first rotation axis 160.

Through this, it is possible to implement a reaction force more accurately.

In this case, the reaction force providing means provided in the encoder of the first sensor 300 and capable of implementing a reaction force may be provided with a motor, and in addition to the motor, it is not limited as long as it is a device that is capable of implementing the reaction force, such as a clutch.

In addition, since the first sensor 300 of the sensing device 2 according to an embodiment of the present disclosure may be arranged on one side from the first side wall member 510 and the second side wall member 520 through the first rotation axis 160 connected to the first rotating body 130, for this purpose, the second side wall member 520 may be provided with a sensor hole 511 for arranging the first sensor 300.

A fixing part 340 of the first sensor 300 may be coupled to the sensor hole 511 to stably support the first sensor 300, and the frame 330 extending along an extension direction of the first rotation axis 160 may be arranged near a corner of the fixing part 340.

In this case, the rotating part 320 of the first sensor 300 coupled to the first rotation axis 160 and rotated together with the first rotation axis 160 may be arranged near a center of the fixing part 340.

Accordingly, the rotating part 320 may be protected by the frame 330 surrounding the rotating part 320 while it rotates.

Meanwhile, the sensing device 2 according to an embodiment of the present disclosure may further include a second handle 210 formed to be rotatable in a different direction from the first handle 110.

The second handle 210 may rotate around a third rotation axis located inside.

The user may control a linear movement of the passive member according to an orbital movement of the first handle 110, and may control the roll direction movement that rotates around a longitudinal central axis of the passive member through the second handle 210.

In this case, the third rotation axis, which is a rotational central axis of the second handle 210, may be arranged to face a different direction from the first rotation axis 160.

For example, the third rotation axis of the second handle 210 may be arranged to extend in an up-down direction, unlike the first rotation axis that extends in a left-right direction as illustrated in FIG. 1.

If the first surface on which the first handle 110 is placed is a plane, the third rotation axis may be parallel to the first surface or belong to the first surface.

For example, as illustrated in FIG. 1, if the first handle 110 is formed in a circular shape, and the first surface is a plane including a circle in which the first handle 110 is formed, the third rotation axis may be included in the first surface and may be arranged perpendicularly to the first rotation axis.

If the passive member is provided in a long tube shape, bar shape or wire shape, in order to control the passive member, the user may control a longitudinal movement of the passive member and a rotational movement with the longitudinal direction of the passive member as an axis.

Such a longitudinal movement of the passive member may be controlled through the first handle 110, and the rotational movement of the passive member may be controlled through the second handle 210.

In this case, in order to remotely control the longitudinal movement and the rotational movement of the passive member similar to an actual sense, the second handle 210 may be provided with a first hole 241 that is penetrated by the first handle 110 in the longitudinal direction.

As a result, as illustrated, the second handle 210 is formed in a pipe shape or a column shape, and at least a portion of the first handle 110 may penetrate the second handle 210 and be placed inside the second handle 210.

Accordingly, the user may implement an action of moving and freely rotating a tubular member in one direction similar to reality through the first handle 110 and the second handle 210.

In this case, since the second handle 210 may be provided in a rotatable cylinder shape, the rotation angle of the second handle 210 is not limited. The second handle 210 may be rotated infinitely without angle restrictions while being penetrated by the first handle 110.

In this case, the driving force according to a rotation of the second handle 210 may be transmitted through the second rotating body 240 that rotates in conjunction with the rotational movement of the second handle 210.

The second rotating body 240 may be directly or indirectly connected to the second handle 210.

For example, the second rotating body 240 may be formed as a gear that is engaged with the gear teeth provided on the second handle 210 and rotates.

Referring to FIGS. 8 and 9, a third rotating body 230 that rotates around a third rotation axis together with the second handle 210 may be arranged below the second handle 210.

The third rotating body 230 may be formed as a circular member surrounding the second handle 210, and may be connected to a second power transmission member 220 in the form of a belt together with the second rotating body 240.

In this case, the third rotating body 230 may perform a function of transmitting the rotational force of the second handle 210 through the second power transmission member 220.

For this purpose, the shape of the third rotating body 230 is not limited, and it may be formed in a pulley shape surrounding the second handle 210, and may also be formed in a gear shape.

The second power transmission member 220 may be formed in a belt shape that covers at least a portion of the periphery of the third rotating body 230 and at least a portion of the periphery of the second rotating body 240, and the second power transmission member 220 is not limited as long as it can transmit the rotational force of the third rotating body 230 to the second rotating body 240.

Depending on the structures of the second handle 210, the second rotating body 240 and the third rotating body 230 formed in this structure, when the second handle 210 rotates together with the third rotating body 230, the second rotating body 240 may be rotated in a predetermined direction by the second power transmission member 220.

For example, when the second handle 210 is rotated clockwise, the third rotating body 230 that rotates as one body with the second handle 210 may also rotate clockwise.

In this case, the second power transmission member 220 may also rotate clockwise according to a clockwise rotation of the third rotating body 230, and the second rotating body 240 connected to the second power transmission member 220 may also rotate clockwise.

The second rotating body 240 may rotate around the second rotation axis 260 that penetrates the center.

In this case, the second sensor 400 for sensing rotation information of the second rotation axis 260 and the second rotating body 240 may be coupled to an end part of the second rotation axis 260.

The second rotation axis 260 may rotate together with the second rotating body 240 at a center of the second rotating body 240.

Accordingly, the second sensor 400 may sense the rotation information of the second rotation axis 260 and sense the rotational movement information of the second rotating body 240, the second power transmission member 220, the third rotating body 230 and the second handle 210.

To this end, the second sensor 400 may be provided with an encoder that can collect information on the rotational movement of the second rotating body 240, such as the rotation amount, angular velocity and torque.

Referring to FIGS. 1 to 10, the second sensor 400 may include a second sensor body 410 and a rotational part 420.

In this case, the second handle 210, the third rotating body 230, the second power transmission member 220, the second rotating body 240 and the second sensor 400 may be relatively arranged on a front side of the sensing device 2 according to an embodiment of the present disclosure.

Accordingly, a front housing 560 that can support and cover the second sensor 400, the second rotating body 240 and the third rotating body 230 may be arranged on a front side of the sensing device 2 according to an embodiment of the present disclosure.

In this case, the front housing 560 may be formed to cover the left and right sides of the second sensor 400, the second rotating body 240 and the second rotation axis 260.

The rotational part 420 of the second sensor 400 may be coupled to the second rotation axis 260 and rotate together with the second rotation axis 260.

As the second rotation axis 260 rotates together with the second rotating body 240, the rotational part 420 is rotated integrally with the second rotating body 240, and information on the rotational movement of the second rotating body 240 may be sensed by an encoder provided in the second sensor body 410.

In this case, an encoder provided with a reaction force providing means may be placed in the second sensor body 410.

Accordingly, the second sensor 400 may transmit a predetermined reaction force toward the second handle 210 by the reaction force providing means provided in the encoder of the second sensor 400.

More specifically, the second sensor 400 may transmit a predetermined torque to the second rotation axis 260, by using the reaction force providing means provided in the encoder of the second sensor 400.

The torque transmitted to the second rotation axis 260 may be transmitted to the second rotating body 240, and the torque transmitted to the second rotating body 240 may be transmitted to the third rotating body 230 or the second handle 210 directly by the second rotating body 240 through the second power transmission member 220 and the third rotating body 230, or if the second power transmission member 220 is not provided, the second rotating body 240 may directly transmit the torque to the third rotating body 230 or the second handle 210.

Accordingly, when the passive member comes into contact with an obstacle within an organ or receives an external force due to the surrounding environment, the external force may be transmitted to the second handle 210. Accordingly, it is possible to implement a reaction force that can be felt when the user directly grips and manipulates the passive member.

In this case, the second sensor 400 may also include a torque sensor that can sense a torque applied to the second rotation axis 260.

Through this, it is possible to implement a reaction force more accurately.

In this case, the reaction force providing means provided in the encoder of the second sensor 400 and capable of implementing the reaction force may be provided with a motor, and in addition to the motor, it is not limited as long as it is a device that is capable of implementing the reaction force, such as a clutch. Meanwhile, referring to FIGS. 1 to 9, the sensing device 2 according to an embodiment of the present disclosure may include a first side wall member 510 and a working surface 540 coupled to the first side wall member 510 at a predetermined angle.

The working surface 540 may be formed such that an upper surface thereof is parallel to the bottom surface 550.

In addition, the working surface 540 may be provided with a second hole 541 formed to penetrate the working surface 540 in an up-down direction.

The second hole 541 may be coupled with a second handle 210 having a first hole 241 therein.

In this case, since the first hole 241 is positioned inside the second hole 541, the first hole 241 and the second hole 541 may be penetrated together by the first handle 110.

In this case, in order for the second handle 210 to be more stably coupled to the working surface 540 through the second hole 541, a separate second handle coupling member may be placed between the second handle 210 and the working surface 540.

Accordingly, the second handle 210 may be more firmly coupled to the working surface 540.

An upper surface of the working surface 540 may be formed as a plane while being parallel to the bottom surface 550.

Accordingly, the user may operate the sensing device 2 while leaning a part of his hand or arm on the working surface 540.

Accordingly, the user may control the sensing device 2 more stably and precisely, and at the same time, it is possible to reduce fatigue even during long-term use of the sensing device 2.

In this case, a first insertion groove 551 and a second insertion groove 553 for coupling the first side wall member 510 and the second side wall member 520 may be formed on the bottom surface 550.

As the first side wall member 510 is inserted into the first insertion groove 551 and the second side wall member 520 is inserted into the second insertion groove 553, the sensing device 2 according to an embodiment of the present disclosure may be supported more firmly.

The sensing device 2 according to an embodiment of the present disclosure having such a structure is provided with a separate switch 700 such that it is possible to control the power of the sensing device 2 or the connection status with the passive member.

Hereinafter, the description will be provided for a modified example of the sensing device 2 according to an embodiment of the present disclosure according to the above structure, and other embodiments, by using different drawings.

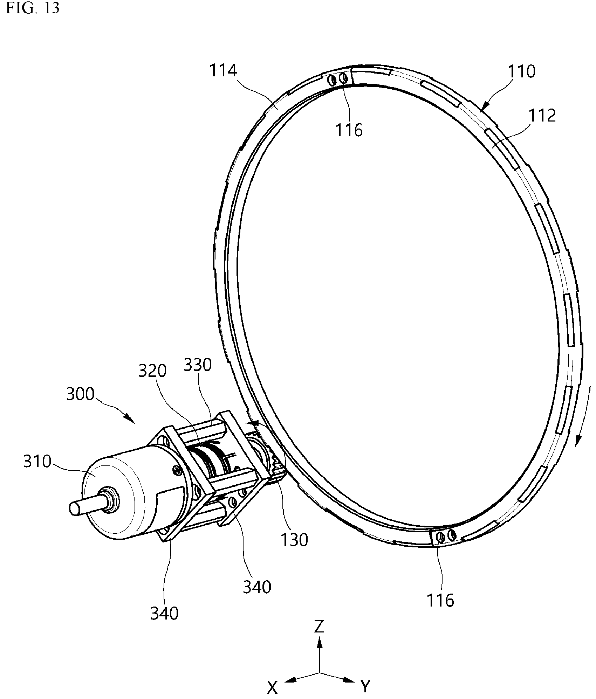

FIG. 13 is a drawing illustrating a first modified example of some configurations related to the rotational movement of a first handle of a sensing device according to an embodiment of the present disclosure.

The first modified example of the sensing device 2 according to an embodiment of the present disclosure, as illustrated in FIG. 13, does not have a first power transmission member 120 connected to the first handle 110.

In this case, the first rotating body 130 may be arranged to contact the first handle 110, and accordingly, the torque according to the rotation of the first handle 110 may be directly transmitted to the first rotating body 130 by a frictional force without the first power transmission member 120.

In this case, a spring may be used to maintain a close contact state between the first handle 110 and the first rotating body 130 by an elastic force.

In this case, the rotation direction of the first rotating body 130 and the rotation direction of the first handle 110 may be opposite to each other.

Referring to FIG. 13, when the first handle 110 rotates clockwise, the first rotating body 130 may rotate counterclockwise by engaging with the first handle 110.

In this case, since the rotational force is directly transmitted from the first handle 110 to the first rotating body 130 without the intervention of the first power transmission member 120, it may be formed with a simpler structure.

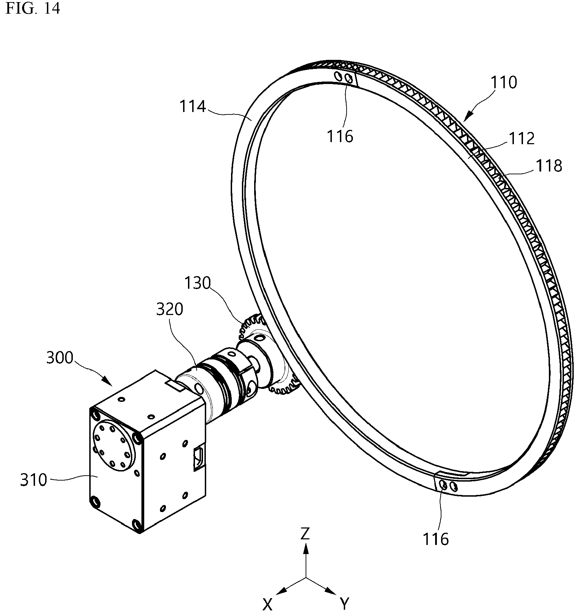

FIG. 14 is a drawing illustrating a second modified example of some configurations related to the rotational movement of a first handle of a sensing device according to an embodiment of the present disclosure.

In this case, as in the case of the first modified example of the sensing device 2 according to an embodiment of the present disclosure, the first rotating body 130 may be arranged to come into contact with the first handle 110, and thus, the torque according to the rotation of the first handle 110 may be directly transmitted to the first rotating body 130 without the first power transmission member 120.

In this case, the first handle 110 may include gear teeth 118 that are continuously provided along a peripheral part.

The first rotating body 130, like the first handle 110, may be provided with a gear including gear teeth that are continuously provided along a peripheral part.

Accordingly, the first rotating body 130 is engaged with the first handle 110, and the first rotating body 130 may rotate together with the rotation of the first handle 110.

In this case, the rotation direction of the first rotating body 130 and the rotation direction of the first handle 110 may be opposite to each other.

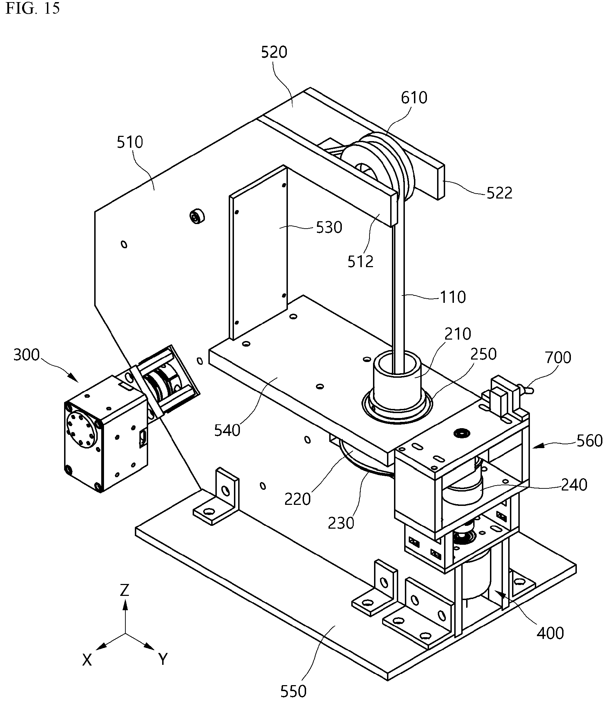

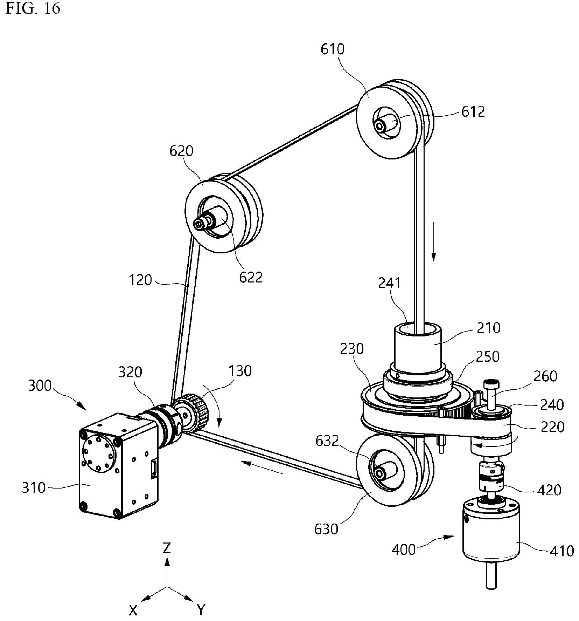

FIG. 15 is a perspective view of a sensing device according to another embodiment of the present disclosure. FIG. 16 is a drawing illustrating some configurations related to the rotational movement of a first handle of a sensing device according to another embodiment of the present disclosure.

Referring to FIG. 15, the sensing device 2 according to another embodiment of the present disclosure may include a first handle 110 formed in a belt shape.

Accordingly, the path of the first handle 110 may be formed as a closed curve including a straight portion.

Referring to FIG. 16, the first handle 110 of the sensing device 2 according to another embodiment of the present disclosure may be supported by the first support member 610, the second support member 620, the third support member 630 and the first rotating body 130.

In this case, unlike the support member in the sensing device 2 according to an embodiment of the present disclosure, which supports both the outer and inner surfaces of the first handle 110, the support member in the sensing device 2 according to another embodiment of the present disclosure may support only the inner surface of the first handle 110.

In this case, when the user manipulates the first handle 110, the first handle 110 may be manipulated in a form that is closer to a linear movement compared to the sensing device 2 according to an embodiment of the present disclosure.

The first handle 110 may be formed of an elastic material, and in this case, it is possible to change the position of the first handle 110 according to the change in the position of the support member.

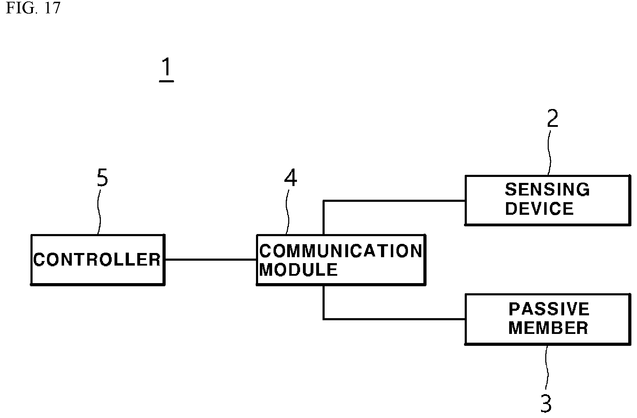

FIG. 17 is a structural diagram of a control system including a sensing device according to an embodiment of the present disclosure.

The sensing device 2 according to an embodiment of the present disclosure is a sensing device that senses information for controlling a passive member 3, and the passive member 3 may be operated by a communication module 4 that can exchange electrical signals between the sensing device 2 and the passive member 3.

In this case, the communication method applied to the communication module 4 may be either wired communication or wireless communication.

In this case, the electrical signals may be controlled by a separate controller 5.

The controller 5 interprets the orbital movement information of the first handle 110 and the rotational movement information of the second handle 210 obtained through the sensing device 2, and accordingly, it may issue a command to the passive member 3.

For example, when the first handle 110 turns clockwise, the passive member 3 may be controlled in a direction of further inserting the passive member 3 into the organ, and when the second handle 110 turns clockwise, the passive member 3 may be turned clockwise.

In this case, the controller 5 may not only handle the movement information obtained through the sensing device 2, but may also handle the information obtained from the passive member 3.

For example, when the passive member 3 comes into contact with a predetermined object within the organ, the reaction force transmitted to the passive member 3 may be transmitted to the sensing device 2 as an electrical signal through the communication module 4 and the controller 5.

The sensing device 2 may accordingly operate motors built into the first sensor 300 and the second sensor 400 to implement a reaction force to the first handle 110 and the second handle 210.

This process may be performed through a pressure sensor included in the passive member 3.

When pressure is sensed, the communication module 4 and the controller 5 may transmit the pressure as a reaction force to the first handle 110 or the second handle 210.

The sensing device 2 according to an embodiment of the present disclosure and a control system 1 including the same, having the structure as described above, are not limited to being used in a tubular surgical tool such as a catheter or guide wire as a passive member 3.

For example, the sensing device 2 according to an embodiment of the present disclosure and the control system 1 including the same may be applied to equipment such as an endoscope that is inserted into a pipe, and the industrial fields to which they can be applied are not limited.

According to this configuration, the sensing device 2 according to an embodiment of the present disclosure and the control system 1 including the same may remotely control a surgical tool in a motion similar to an motion of actually manipulating a surgical tool by manipulating a linear movement of the surgical tool by turning the first handle 110 in the form of a closed curve and manipulating a rotational movement of the surgical tool by turning a second handle 210 in the form of a cylinder surrounding the first handle 110.

In addition, the sensing device 2 according to an embodiment of the present disclosure and the control system 1 including the same may control the linear movement through the first handle 110, thereby controlling the linear movement as a continuous movement without displacement limitation.

In addition, the sensing device 2 according to an embodiment of the present disclosure and the control system 1 including the same are provided such that the second handle 210 in charge of the rotational movement surrounds the first handle 110 in charge of the linear movement, and thus, it is easy to control the linear movement and the rotational movement simultaneously.

In addition, the sensing device 2 according to an embodiment of the present disclosure and the control system 1 including the same adopt a motor and a torque sensor inside the first sensor 300 and the second sensor 400 such that the reaction force applied to the surgical tool can be transmitted to the handle, and thus, the reaction force that can be applied when actually operating the surgical tool may be transmitted similarly.

In addition, the sensing device 2 according to an embodiment of the present disclosure and the control system 1 including the same position the first handle 110 and the second handle 210 on the working surface 540 such that the user can perform long-term remote control with minimal fatigue.

Although one embodiment of the present disclosure has been described above, the spirit of the present disclosure is not limited to the embodiment presented in the present specification, and those skilled in the art who understand the spirit of the present disclosure will be able to easily suggest other embodiments by modifying, changing, deleting or adding components within the scope of the same spirit, but this will also be considered to fall within the spirit of the present disclosure.

This invention was derived as part of the following national research project:
[Project Unique ID] 1711186013
[Project Number] 2022R1A2C200698611(2204031)
[Government Department] Ministry of Science and ICT
[Funding Agency] National Research Foundation of Korea
[Research Project Name] Mid-Career Researcher Program
[Research Topic] Remote control technology using flexible endoscopic robot and AI for endoscopic retrograde cholangiopancreatography (ERCP)
[Research Institution] Korea University Research and Business Foundation
[Research Period] March 1, 2022 ~ February 28, 2026

## Claims

1. A sensing device, comprising:
a housing comprising a first side wall member arranged in an up-down direction with respect to a bottom surface;
a first handle formed in a closed curve shape, at least a portion of which is exposed to an outside of the housing and placed on a predetermined first surface, and coupled to one side of the first side wall member so as to be capable of orbital movement along an extension direction of the closed curve at a predetermined location on the closed curve;
a first rotating body configured to rotate around a predetermined first rotation axis in conjunction with an orbital movement of the first handle;
a support member coupled to the housing, and configured to support the first handle such that the first handle maintains a fixed position at the predetermined location; and
a first sensor configured to sense information on a rotational movement of the first rotating body.

2. The sensing device of claim 1, further comprising:
a second handle formed to be rotatable at a fixed position;
a second rotating body configured to rotate around a predetermined second rotation axis in conjunction with a rotational movement of the second handle; and
a second sensor configured to sense information about a rotational movement of the second rotating body.

3. The sensing device of claim 2, wherein the first surface is a plane, and the second rotation axis is parallel to the first surface or belongs to the first surface.

4. The sensing device of claim 3, wherein the second handle is formed in a pipe shape comprising a first hole penetrating the second handle in a direction parallel to the second rotation axis, and at least a portion of the first handle penetrates the first hole.

5. The sensing device of claim 2, 3 or 4, further comprising:
a third rotating body configured to share a rotation axis with the second handle and be connected to a lower part of the second handle and rotate integrally with the second handle,
wherein the second rotating body is rotated by a rotational force of the third rotating body.

6. The sensing device of claim 5, wherein the third rotating body rotates around a third rotation axis parallel to the second rotation axis,
wherein a second power transmission member in a belt shape is connected to a peripheral part of the third rotating body and a peripheral part of the second rotating body, and
wherein the second power transmission member transmits a rotational force of the third rotating body to the second rotating body.

7. The sensing device of claim 6, wherein the second sensor is coupled to the second rotation axis, and senses information about a rotational movement of the second rotating body transmitted through the second rotation axis.

8. The sensing device of claim 6 or 7, further comprising:
a plate-shaped working surface configured to cross the first side wall member at a predetermined height,
wherein the working surface is formed with a second hole penetrated by the second handle, and to which the second handle is coupled.

9. The sensing device of claim 8, wherein the second rotation axis is received at one end part of the working surface, and a receiving groove for supporting the second rotation axis is formed by being recessed in a direction perpendicular to an axial direction of the second rotation axis.

10. The sensing device of any one of claims 2 to 9, wherein the first surface is a plane, and the first rotating body is arranged on the first surface,
wherein a peripheral part of the first handle and an outer surface of the first rotating body are connected together to a first power transmission member in a belt shape, and
wherein the first power transmission member transmits a driving force according to an orbital movement of the first handle to the first rotating body to rotate the first rotating body.

11. The sensing device of claim 10, wherein the first sensor is coupled to a first rotation axis that becomes a center of rotation of the first rotating body,
wherein the first sensor comprises a reaction force providing means for rotating the first rotation axis, and
wherein the first sensor is capable of transmitting a force toward the first handle through the first power transmission member by rotating the first rotating body.

12. The sensing device of any one of claims 2 to 11, wherein a peripheral part of the first handle and an outer surface of the first rotating body are arranged to be in contact with each other.

13. The sensing device of any one of claims 2 to 12, wherein the first handle is formed in a belt shape, and
wherein an outer surface of the first rotating body is formed to contact an inner surface of the first handle such that the first rotating body is rotated according to an orbital movement of the first handle.

14. The sensing device according to any one of claims 12 to 13, wherein the first sensor is coupled to a first rotation axis that becomes a center of rotation of the first rotating body,
wherein the first sensor comprises a reaction force providing means for rotating the first rotation axis, and
wherein the first sensor is capable of transmitting a force toward the first handle by rotating the first rotating body.

15. The sensing device of any one of claims 7 to 14, wherein the second sensor comprises a reaction force providing means for rotating the second rotation axis, and
wherein the second sensor is capable of transmitting a force toward the second handle through the second power transmission member by rotating the second rotational body.
